# EUROPEAN PATENT APPLICATION

(11) **EP 0 804 902 A2**
(43) Date of publication of application: **05.11.1997**
(21) Application number: 97202079.6
(22) Date of filing: 29.05.1992
(51) Int. Cl.: A61B 17/02

(54) **Endoscopic inflatable retraction devices and method of making**

(30) Priority: 29.05.1991 US 706781; 19.11.1991 US 794590
(62) Divisional of application: 92912904.7
(71) Applicant: ORIGIN MEDSYSTEMS, INC., Menlo Park, CA 94025 (US)
(72) Inventor: Moll, Frederic H., San Francisco, CA 94118 (US); Chin, Albert K., Palo Alto, CA 94303 (US); Gadacz, Thomas R., Augusta, CA 30909 (US)
(74) Representative: Schmitz, Jean-Marie

(57) **Abstract**

The present invention provides an apparatus (3) for providing a retraction force from outside the body to retract first tissue (e.g. L) inside the body to gain access to adjacent tissue (e.g. GB), comprising inflatable chamber means (8) for engaging the first tissue and a shaft means (13) for manipulating the chamber means, the inflatable chamber means (8) being defined by an envelope (18) and the envelope is rolled when the chamber means is in its collapsed state.

## Description

The present invention concerns an apparatus for providing a retraction force from outside the body to retract first tissue inside the body to gain access to an adjacent tissue according to the precharacterizing portion of claim 1.

The invention relates to devices for use in laparoscopic surgery, in particular, to devices that provide retraction of an organ to gain access to treat or observe a tissue.

Laparoscopy dates back to the turn of the 20^{th} Century. Early laparoscopic techniques were used primarily for diagnostic purposes to view the internal organs, without the necessity of conventional surgery. Since the 1930s laparoscopy has been used for sterilization and, more recently, for the suturing of hernias. US-A-4,919,152 and 4,944,443 are concerned with techniques for suturing hernias. Another very recent innovation is the use of laparoscopic surgery for removing the gallbladder.

Published International Application No. WO-A-92/21294 under the Patent Cooperation Treaty, describes an apparatus and method wherein the abdominal wall is lifted away from the underlying abdominal organs by an inflatable device which is introduced laparoscopically and, once in place, inflated to engage and lift an extensive area of the abdominal wall.

Even when such lifting techniques are used, it is still necessary to retract other organs to gain access to the organ or tissue to be treated or observed. In other procedures, to gain access to the organ or tissue to be treated or observed, it is necessary to separate the organ to be treated from tissue surrounding it. For example, to be able to observe the outer surface of the heart, the outer surface of the heart has to be separated from the pericardium.

Published International Application No. WO-A 92/21293 under the Patent Cooperation Treaty, describes inflatable retraction devices that retract organs or tissues by means of an inflatable chamber. The retraction device is introduced in a collapsed state into the body through a small incision, and, once in place, inflated to engage an extensive area of the organ or tissue to be retracted, and to gently retract or displace the organ or tissue without damaging it. During laparoscopic treatment and observation procedures, the retraction device retains its expanded condition, and hence its ability to provide retraction, while providing access for surgical instruments through itself to the organ or tissue being treated or observed, or allowing an organ or tissue to be brought inside itself for observation or treatment.

The inflatable retraction devices described in WO-A 92/21293, and one of the inflatable retraction devices described in the present application, are placed in the body through a small incision, and, when inflated, retract the organ by pushing against other adjacent organs and tissues. The force exerted by the retraction device against other organs or tissues within the body cavity can sometimes cause trauma and even damage to the other organs. Hence, it is sometimes preferable to provide a retraction device that can be introduced into the body through a small incision, and which provides retraction without exerting a force against other adjacent organs and tissues.

The publication DE-A-2 847 633 describes an apparatus for use inside a body comprising an inflatable chamber means for applying a force between a first organ and a second organ when in an inflated state, the inflatable chamber means being insertable in collapsed state, and an inflating means for inflating the inflatable chamber means when the latter is in place within the body.

To provide a retraction from outside the body, current laparoscopic procedures use several small metal or plastic retractors inserted though a plurality of incisions. The retractors are fixed to a suitable bar to hold them in place once the desired amount of retraction has been achieved. Because such retractors have a relatively small surface area (the retractors have to be small enough to fit through a small incision), they tend to damage and/or cause trauma to the retracted organs. Moreover, the required plurality of incisions in the body wall undoes some of the advantage of using laparoscopic techniques.

The apparatus for providing a retraction force from outside the body to retract a first tissue is defined in the characterizing portion of claim 1.

The present invention relates to inflatable retraction devices that mechanically retract organs and tissues to provide access to treat or observe other organs or tissues. In the following description, the word "organ" will be used to mean an organ or a tissue that is retracted by the retraction device. The word "treat" will be used to mean both treat and observe, and the word "treatment" will be used to mean both treatment and observation. The word "tissue" or the phrase "tissue to be treated" will both be used to mean the organ or the tissue that is treated after the organ has been retracted.

The inflatable retraction device according to the invention provides a retraction force from outside the body to retract an organ inside the body to gain access to an adjacent tissue. The inflatable retraction device comprises an inflatable chamber that engages with the organ, and a shaft for manipulating the inflatable chamber to retract the organ. The shaft has a distal end to which the inflatable chamber is attached, a bore that communicates with the inflatable chamber and allows the inflatable chamber to be inflated to an expanded state when the inflatable chamber is in place within the body. The shaft has a proximal end, which remains outside the body when the inflatable chamber is in place in the body. The inflatable chamber is flat, and substantially oblong.

In a second method according to the invention for retracting, by means of a retraction force provided from outside the body, an organ in the body in the course of treating an adjacent tissue, an inflatable retractor is provided having an inflatable chamber, and a hollow shaft having a distal end and a proximal end. The inflatable chamber is attached to the distal end of the shaft in a collapsed state. A small incision is made in the body. The proximal end of the shaft is manipulated to pass the inflatable chamber and part of the shaft into the body through the small incision, and to place the inflatable chamber adjacent to the organ. A fluid is passed through the shaft to inflate the inflatable chamber into an expanded state. Finally, the proximal end of the shaft is manipulated to engage the organ with the inflatable chamber, and to retract the organ.

In order that the invention may be fully understood, reference is made on the accompanying drawings, wherein
Figure 1A shows an inflatable paddle retractor according to the invention that provides a retraction force from outside the body cavity and has a substantially flat inflatable chamber.
Figure 1B shows one configuration of inflatable chamber of the inflatable retraction device shown in figure 1A.
Figure 1C shows one alternative configuration of the inflatable chamber of the inflatable retraction device shown in figure 1A.
Figure 1D shows details of the shaft of the inflatable retraction device shown in figure 1A.
Figure 1E shows details of the inflation couplet of the inflatable retraction device shown in figure 1A.
Figure 1F shows the sheath for the inflatable retraction device shown in figure 1A.
Figure 1G shows the inflatable chamber of the inflatable retraction device shown in figure 1A retained in its rolled state by detachable lacing.
Figure 1H is a cross-section of the inflatable chamber shown in figure 1B, showing tacking connecting opposite faces of the envelope.
Figure 2A through 2D show a longitudinal cross-section of the abdomen with an inflatable paddle retractor according to the invention, wherein:

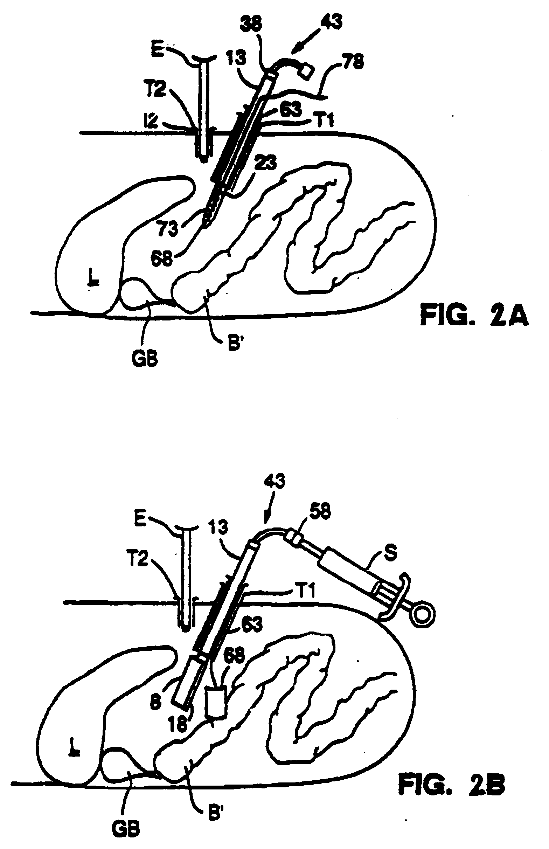

   Figure 2A shows the inflatable paddle retractor after insertion adjacent to the liver.
   Figure 2B shows the inflatable paddle retractor in its expanded state in contact with the liver.

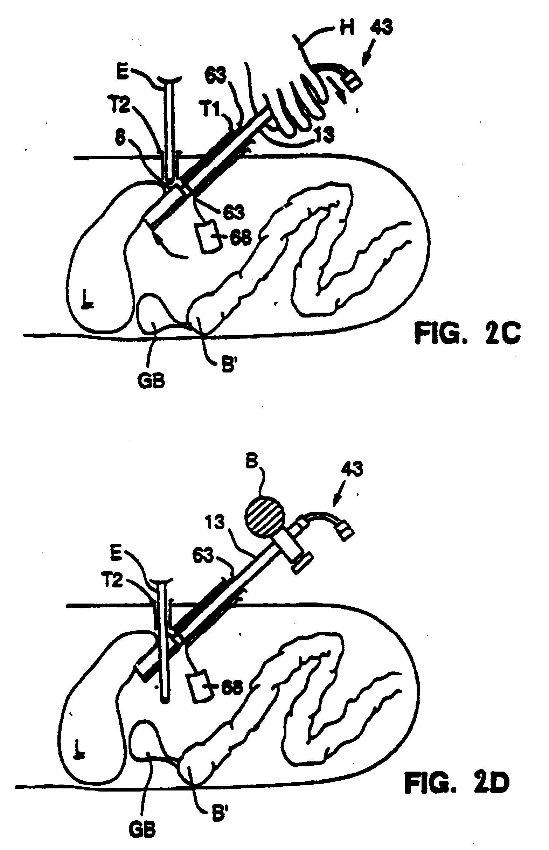

   Figure 2C shows the inflatable paddle retractor being manipulated to retract the liver.
   Figure 2D shows the inflatable paddle retractor clamped to a bar to maintain the liver in its retracted position.

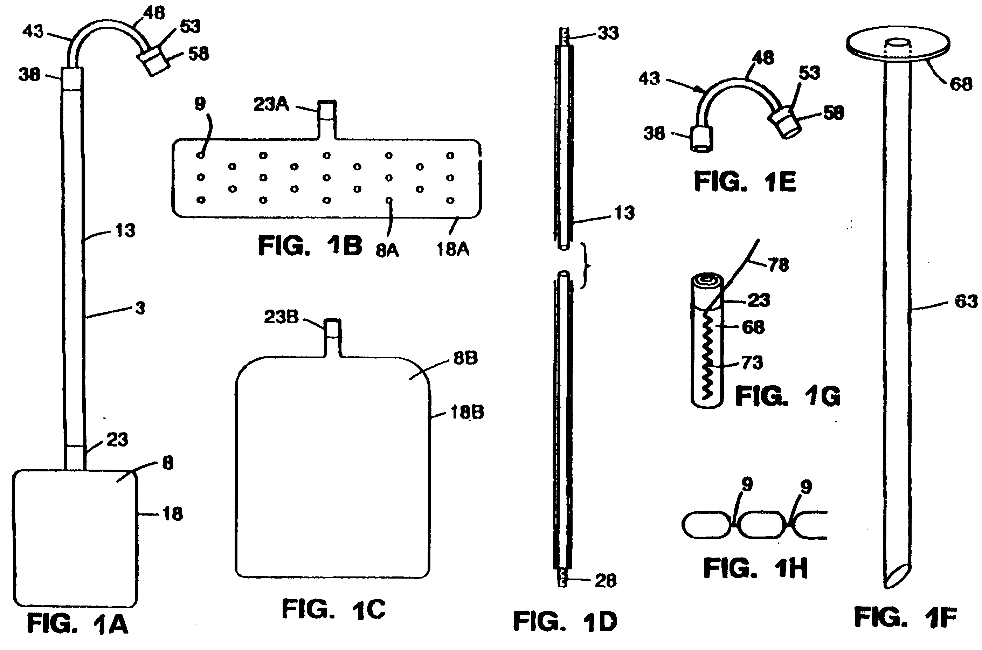

In some procedures, of the prior art the substantial spherical shape of the inflatable retraction devices causes them to obstruct access to the tissue to be treated. The paddle retractor 3 of the present invention shown in figure 1A has a considerably more compact inflatable chamber than a substantially spherical inflatable retraction device. The more compact inflatable chamber provides a large surface area to engage the organ but is less likely to obstruct access to the tissue. The paddle retractor is inserted into a part of the body, such as the abdomen, through a trocar inserted into a single small incision about 10-20 mm long in the body wall.

The paddle retractor 3 has a flat, rectangular, inflatable chamber 8 attached to a hollow shaft 13. The inflatable chamber 8 is enclosed by an envelope 18 made of molded cellophane, about 0.05 to 0.125 mm (0.002" to 0.005") thick. Other materials that are capable of being collapsed into a relatively small volume but which conform to a molded shape when inflated can be used. Figure 1B shows a version of the inflatable chamber 8A having a length of about 80 mm (3.2") and a width of about 20mm (0.8"). The thickness of the inflatable chamber is about 5 mm (0.2"). Opposite faces of the envelope 18A may be tacked together with the tacks 9, and as additionally shown in the cross-sectional view in figure 1H, to prevent the inflatable chamber 8A of the paddle retractor from ballooning out when inflated.

An alternative inflatable chamber 8B is shown in figure 1C. This inflatable chamber has a length of about 60 mm (2.4") a width of about 50 mm (2"), and a thickness of about 5 mm (0.2°). The alternative inflatable chamber may have the same construction as the inflatable chamber shown in figure 1B.

Returning to figure 5A, the envelope 18 is attached to the coupler 23, preferably by welding. The coupler 23 is made of metal, preferably stainless steel, is hollow, and internally threaded, as can be seen in figure 1C..

The threads in the coupler 23 accept the external threads 28 on the distal end of the shaft 13 (figure 1D).

The shaft 13, shown in detail in figure 1D, has an internal bore 93 about 2..5 mm (0.1") in diameter. The shaft 13 has an external diameter of about 4.5 mm (0.18") and a length that depends on the application. The range of lengths is from about 150 mm (6"), for use in upper abdominal operations, to about 300 mm (12"), which reach to the pelvis for carrying out such operations as appendectomy. The proximal end of the shaft 13 also carries external threads 33.

The external threads 33 of the shaft 13 are screwed into a coupler 38, which forms part of the inflation adapter 43, shown in detail in figure 1E. The coupler is similar to the coupler 23, and has one end of a short length of 3 mm (0.12") outside diameter plastic tubing 48 attached to it. The plastic tubing is preferably polyethylene, and the preferred attachment method is welding. Similarly attached to the other end of the plastic tube is a reflux valve 53, which includes the coupler 58, suitable for accepting a syringe (not shown).

The shaft 13 may be attached directly to the envelope 18 of the inflatable chamber 8 and to the inflation adapter 43, instead of via the couplers 23 and 38. However, it is preferred that the shaft 13 be detachable from the envelope 18 and the inflation adapter 43, which necessitates using the couplers 23 and 38. This way, the envelope 18 and the inflation adapter 43 can be disposable and the shaft 13 and the sheath 63 can be re-sterilized for further use.

The sheath 63 has an external diameter of about 5.5 mm (0.22"), an internal diameter of about 4.5 mm (0.18") and a length of about 150 mm (6"). The proximal end of the sheath 63 is fitted with the flange 88, which is about 25 mm (1") in diameter. The sheath fits inside a standard 5.5 mm internal diameter trocar and projects about 2.5 mm (0.1") beyond its end.

### Method of Using a Paddle Retractor

A method of using a paddle retractor to lift the liver so that the gall bladder can be observed will now be described.

The paddle retractor 3 is supplied with the envelope of the main inflatable chamber in a rolled state such that it forms a linear extension of the coupler 23, as shown in figure 1G. The envelope is retained in its rolled state by the sleeve 68 and the detachable lacing 73. Alternatively, a sleeve with a tear strip, or detachable lacing alone, can be used.

An incision I1 is made in the abdominal wall AW and a 5.5 mm external diameter trocar T1 is driven through the abdominal wall. The sheath 63 is then inserted into the trocar T1. A second incision I2 is made so that a suitable additional trocar T2 can be inserted into the abdomen. An endoscope E is inserted through the trocar T2 to observe the retraction procedure.

The inflatable retraction device is assembled by attaching the couplers 23 and 38 to the distal and proximal ends, respectively, of the shaft 13. The cord 78 attached to the detachable lacing 73 is run along the length of the shaft 13.

The proximal end of the shaft 13 is then grasped and manipulated to insert the rolled envelope 18 and the distal end of the shaft into the abdomen through the sheath 63. Once the rolled envelope 18 has passed through the sheath 63, the proximal end of the shaft 13 is manipulated to bring the rolled envelope close to the liver, as shown in figure 6B. The shaft 13 is then temporarily clamped in position by attaching it to a suitable bar.

The cord 78 is pulled to detach the detachable lacing 73 from the sleeve 68. This releases the sleeve from around the rolled envelope 18. The cord and detachable lacing remain attached to the sleeve so that the sleeve can be withdrawn from the abdominal cavity through the sheath 63 either immediately or at the end of the operation.

A large syringe S, approximately 50 ml, is filled with an inflation fluid and attached to the coupler 58. The preferred inflation fluid is air, although a different gas, such as carbon dioxide, or a liquid, such as saline solution, can be used. The syringe is then operated to drive the inflation fluid through the reflux valve 53, the tube 48, and the shaft 13, into the inflatable chamber 8, as shown in figure 2B. This unrolls the envelope 18 and inflates the inflatable chamber 8. When the inflatable chamber is fully inflated, the syringe is detached from the coupler 58. The reflux valve closes automatically and maintains the inflation pressure in the inflatable chamber.

The proximal end of the shaft 13 is gripped, the shaft 13 is detached from the bar B, and, while observing through the endoscope E, the proximal end of the shaft 13 is manipulated to engage the inflated inflatable chamber 8 with the liver L. The shaft 13 is then further manipulated to push the inflatable chamber against the liver. The force applied to the liver by the relatively large area of the inflatable chamber gently retracts the liver, as shown in figure 2C, so that the gall-bladder GB can be seen through the endoscope E. When liver is suitably retracted, the shaft 13 is once more clamped to the bar B, as shown in figure 2D.

After observation has been completed, the proximal end of the shaft 13 is once more gripped and the shaft is released from the bar. The shaft is then manipulated to disengage the inflatable chamber 8 from the liver and to allow the liver to return to its normal, non-retracted position. The syringe, in its empty position, is reconnected to the coupler 58, which opens the reflux valve 53. The syringe is operated to aspirate the inflation fluid from the inflatable chamber 8. The envelope 18 returns to its rolled position, which enables the proximal end of the shaft 13 once more to be manipulated to withdraw the inflatable retraction device from the abdominal cavity through the sheath 63. The cord 78, detachable lacing 73, and sheath 63 are withdrawn from the abdominal cavity through the sheath 73 by pulling on the cord 78. Finally, the sheath 63 is withdrawn from the trocar T1.

## Claims

1. Apparatus (3) for providing a retraction force from outside the body to retract first tissue (e.g. L) inside the body to gain access to adjacent tissue (e.g. GB), said apparatus comprising inflatable chamber means (8) for engaging the first tissue and shaft means (13) for manipulating the chamber means including a distal end (23) attached to the chamber means, bore means communicating with the chamber means to inflate the chamber means to an expanded state when in place with the body and a proximal end (38) for disposition outside the body when the chamber means is in place within the body; characterized in that the inflatable chamber means (8) is defined by an envelope (18) and the envelope is rolled (Fig. 1G) when the chamber means is in its collapsed state.

2. Apparatus according to claim 1, characterized as further comprising detachable means (68) for maintaining the envelope (18) in its rolled state, said detachable means being detached before the chamber is inflated to its expanded state.

3. The apparatus of claim 1 characterized in that the inflatable chamber means (8) is flat, and substantially oblong.

4. The apparatus of claim 1 characterized in that the inflatable chamber means (8) has a width, a length, and a thickness, and the thickness of the inflatable chamber means is less than one tenth of the longer of the width or the length of the inflatable chamber.

5. The apparatus of claim 2 characterized in additionally comprising a reflux valve means (53), attached to the proximal end of the shaft means (13), for maintaining the inflatable chamber means (8) in its inflated state.
